Europäisches Patentamt

(19) European Patent Office

Office européen des brevets

(11) Publication number: **0 041 778**
**B2**

(12) **NEW EUROPEAN PATENT SPECIFICATION**

(45) Date of publication of the new patent specification:
**24.08.88**

(51) Int. Cl.⁴: **C 07 C 63/26,** C 07 C 63/24,
C 07 C 51/265

(21) Application number: **81302122.7**

(22) Date of filing: **13.05.81**

(54) **Oxidation of meta- or para-xylene to iso- or tere-phthalic acid.**

(30) Priority: **10.06.80 GB 8018918**

(43) Date of publication of application:
**16.12.81 Bulletin 81/50**

(45) Publication of the grant of the patent:
**05.09.84 Bulletin 84/36**

(45) Mention of the opposition decision:
**24.08.88 Bulletin 88/34**

(84) Designated Contracting States:
**BE DE FR GB IT NL**

(56) References cited:
**BE - A - 857 068**
**CH - A - 365 060**
**GB - A - 762 803**
**GB - A - 942 859**
**GB - A - 1 542 320**
**GB - A - 2 019 395**
**GB - A - 2 032 432**
**US - A - 2 833 816**
**US - A - 2 907 792**
**US - A - 3 406 196**

(73) Proprietor: **IMPERIAL CHEMICAL INDUSTRIES PLC,**
**Imperial Chemical House Millbank, London SW1P 3JF**
**(GB)**

(72) Inventor: **Norval, Stephen Vynne, 3 Merlin Close,**
**Guisborough Cleveland (GB)**

(74) Representative: **Taylor, Michael et al, Imperial Chemical**
**Industries PLC Legal Department: Patents Po Box 6,**
**Welwyn Garden City Herts, AL7 1HD (GB)**

EP 0 041 778 B2

## Description

The present invention relates to the oxidation of meta- or para-xylene to iso- or tere-phthalic acid respectively.

Iso- and tere-phthalic acids are chemical intermediates widely used in resin and polymer manufacture. They have been produced for some years by oxidation with molecular oxygen of the corresponding xylene usually in the presence of a catalyst, commonly a heavy metal e.g. cobalt and/or manganese, compound. In addition the heavy metal catalyst may be promoted by elemental bromine or a bromine-containing compound.

The heavy metal/bromine catalysed oxidation is carried out in a solvent which is substantially acetic acid containing up to 20% by weight water. The use of this solvent while necessary to ensure the solubility of reactants and catalysts has the pronounced disadvantage that, under the conditions of the oxidation, the acetic acid is itself oxidised and has to be replaced, with a consequent increase in process cost. The obvious solvent to use in place of the acetic acid is water which is cheap, not oxidised and less corrosive but the solvent capability of water alone for the xylene reactant is inadequate so that poor yields are obtained. We have now carried out investigations into the use of water as a solvent and we have devised a method of oxidising meta- or para-xylene in a predominantly aqueous reaction medium which gives a better yield or purer iso- or tere-phthalic acid.

Accordingly, the invention is a continuous process for the production of iso- or tere-phthalic acid which comprises oxidising by means of molecular oxygen meta- or para-xylene in the presence of a catalyst comprising bromine or a bromine-containing compound alone or together with a heavy metal catalyst, characterised in that the reaction is carried out in an essentially aqueous reaction medium and is initiated by adding, on start-up only, a solubilising agent for the meta- or para-xylene.

For convenience, in the remainder of this specification the invention will be described in terms of the oxidation of p-xylene to tere-phthalic acid. It should be understood however that the description applies mutatis mutandis to the oxidation of meta-xylene to isophthalic acid.

The solubilising agent for the oxidation of p-xylene is terephthalic acid, the product of the process. We have found that, although tere-phthalic acid is formed as the oxidation process proceeds, unless there is sufficient present ab initio the rate of formation is insufficient to provide enough to exert an adequate initial solubilising effect. At the temperature at which the oxidation is carried out the solubility of the tere-phthalic acid is in the range 1 to 20% by weight of the aqueous reaction medium and it is preferred that such a concentration of tere-phthalic acid should be present at the start of the oxidation.

The process according to the invention is carried out at a temperature in the range 200 to 300°C preferably 210 to 260°C i.e. higher than the temperature generally used in the prior art processes. The pressure under which the process is conducted is at least such that a liquid phase is maintained in the reactor and is, for example, up to 250 bar preferably 15 to 150 bar.

The molecular oxygen used in the process may be used alone or in admixture with other gases e.g. as air or as a mixture of oxygen and nitrogen with a higher or lower oxygen content than that of air.

The catalyst is bromine or a bromine-containing compound which may be used alone or together with a heavy metal catalyst. The bromine may be provided as bromine itself, as hydrogen bromide, as an organic bromine compound e.g. tetrabromoethane or as an inorganic bromide. Suitable bromides include, for example, bromides of the heavy metals when the latter are present, for example manganese or cobalt bromides. The amount of bromine present in the process may vary widely but it generally lies in the range 500 to 50,000 ppm based on the weight of solvent. When used with a heavy metal it is preferred that the amount of bromine is in excess of the stoichiometric amount required to form a salt with the metal, especially up to 20:1 bromine: heavy metal (atomic ratio). Preferably the bromine is provided as hydrogen bromide.

The heavy metals which may be used as catalysts include vanadium, chromium, manganese, iron, cobalt, nickel, molybdenum, a lanthanide e.g. cerium, hafnium and zirconium. Particularly suitable is manganese especially in combination with cobalt and/or nickel. The amount of heavy metal present during the oxidation may vary widely. When manganese and cobalt are the heavy metals the concentration of manganese may be 20 to 20,000 ppm based on solvent and cobalt 10 to 10,000 ppm, the manganese being in excess. Preferably the manganese is present in up to 20 times atomic excess of any other heavy metal present. The heavy metals may be used, for example, in the form of their inorganic or organic acid salts especially the bromides or lower ($C_1$ to $C_4$) alkanoates e.g. acetates.

It is also possible to use as catalyst promoter iodine or an iodine-containing compound. The iodine-containing compound may be organic e.g. iodide or inorganic. Preferred inorganic iodine-containing compounds include hydrogen iodide, alkali metal iodides e.g. lithium, sodium and potassium iodide and ammonium iodide. The iodine may also be provided in the form of the iodide of the heavy metal used as catalyst in the process e.g. as manganese and/or cobalt iodide. The amount of iodine or iodine-containing compound present in the process is preferably in the range 0.1 to 1,000 ppm based on the weight of solvent, more preferably 1 to 500 ppm.

The separation and purification of the tere-phthalic acid product of the process may be carried out by known means. Thus the product may be cooled, separated by filtration or by centrifuging and washed with fresh solvent. It may also be subject to a secondary or postoxidation to improve its quality and/or it may be catalytically hydrogenated in the presence of a catalyst e.g. platinum to achieve same object.

The invention will now be further described with reference to the following Examples in which the apparatus used consisted of a titanium autoclave od total capacity 4.5 litres equipped with a heater, reflux condenser, agitator, gas inlet and outlet, and feed inlet and outlet.

## Example 1

The autoclave was charged with 1800 ml water, 10.0 g of manganese dibromide tetrahydrate, 1.88 g of cobalt dibromide hexahydrate 0.12 g potassium iodide, 13.2 ml of 47% by wight aqueous HBr and 90 g of purified tere-phthalic acid. The reactor contents were heated to 230°C at a total pressure of 38 bar under a nitrogen gas flow. A flow of approximately 20% oxygen in nitrogen was then substituted for the nitrogen flow and 130 ml p-xylene introduced continuously to the reactor over a period of one hour. A sample of the reaction mixture was then removed from the reactor. The p-xylene feed to the reactor having ceased, the oxygen and nitrogen flow was continued for a further 30 minutes while the temperature was maintained at 230°C. Another sample of reaction mixture was then removed from the reactor. The samples of reaction mixture were cooled to ambient temperature and the product filtered, washed with water and dried. The solid tere-phthalic acid products were found to contain 1.8% by weight 4-carboxybenzaldehyde (4CBA) and 1.5% by weight p-toluic acid (pTA) following the initial reaction period and 1.0% by weight 4CBA and 0.64% by weight pTA following the further 30 minute oxidation. (4CBA and pTA are intermediate oxidation products in the conversion of p-xylene to tere-phthalic acid).

## Example 2

The preceding experiment was repeated, excepting that no tere-phthalic acid was charged to the autoclave. Little oxygen was taken up in the reactor, indicating a very slow oxidation reaction and the product obtained was a dark brown tar-like solid.

## Example 3

The experiment of Example 1 was repeated, excepting that the reactor was heated to 248°C at a total pressure of 56 bar and the p-xylene flow to the reactor was 62 ml over 1 hour. Following the initial reaction period, the solid tere-phthalic acid product was found to contain 0.4% by weight 4CBA and 0.4% by weight pTA. Following a further 30 minutes oxidation, the entire reactor contents were cooled to about 150°C under nitrogen flow and the solid tere-phthalic acid product filtered off, washed with water and dried. This product was found to contain 0.05% by weight 4CBA and 0.10% by weight pTA. A total of 164 g TA was recovered, indicating a yield of 89% of the p-xylene fed to the reactor.

## Example 4

The experiment of Example 2 was repeated, excepting that the reactor was heated to 245°C at a total pressure of 56 bar and the p-xylene flow to the reactor was 180 ml over 1 hour. Little oxygen uptake was observed and no crystalline products were obtained.

## Claims

1. A continuous process for the production of iso- or terephthalic acid which comprises oxidising by means of molecular oxygen meta- or para-xylene in the presence of a catalyst comprising bromine or a bromine-containing compound alone or together with a heavy metal catalyst characterised in that the reaction is carried out at a temperature in the range 200 to 300°C in an essentially aqueous reaction medium and is initiated by adding, on start-up only, iso- or terephthalic acid respectively as a solubilising agent for the meta-or para-xylene.

2. A process according to Claim 1, characterised in that the reaction is initiated in the presence of 1 to 20% by weight of the aqueous reaction medium of isophthalic or terephthalic acid.

3. A process according to any preceding claim characterised in that it is carried out at a temperature in the range 210°C to 260°C.

4. A process according to any one of the preceding claims characterised in that the heavy metal is manganese optionally together with cobalt and/or nickel.

5. A process according to any one of the preceding claims characterised in that bromine, hydrogen bromide or a bromide of a heavy metal is present.

6. A process according to any one of the preceding claims characterised in that iodine or an iodine-containing compound is present as catalyst promoter.

## Patentansprüche

1. Kontinuierliches Verfahren zur Herstellung von Iso- oder Terephthalsäure, bei dem m- oder p-Xylol in Gegenwart eines Katalysators, der Brom oder eine bromhaltige Verbindung allein oder zusammen mit einem Schwermetallkatalysator enthält, durch molekularen Sauerstoff oxidiert wird, dadurch gekennzeichnet, dass die Reaktion bei einer Temperatur im Bereich von 200 bis 300°C in einem im wesentlichen wässrigen Reaktionsmedium durchgeführt wird und eingeleitet wird, indem nur beim Anfahren Iso- bzw. Terephthalsäure als Löslichkeitsvermittler für das m- oder p-Xylol zugegeben wird.

2. Verfahren nach Anspruch 1, dadurch gekennzeichnet, dass die Reaktion in Gegenwart von 1 bis 20 Masse% Isophthalsäure oder Terephthalsäure, auf die Masse des wässrigen Reaktionsmediums bezogen, eingeleitet wird.

3. Verfahren nach einem der vorhergehenden Ansprüche, dadurch gekennzeichnet, dass es bei einer Temperatur im Bereich von 210°C bis 260°C durchgeführt wird.

4. Verfahren nach einem der vorhergehenden Ansprüche, dadurch gekennzeichnet, dass das Schwermetall Mangan gegebenenfalls zusammen mit Cobalt und/oder Nickel ist.

5. Verfahren nach einem der vorhergehenden Ansprüche, dadurch gekennzeichnet, dass Brom, Bromwasserstoff oder ein Bromid eines Schwermetalls vorhanden ist.

6. Verfahren nach einem der vorhergehenden Ansprüche, dadurch gekennzeichnet, dass als Promotor für den Katalysator Iod oder eine iodhaltige Verbindung vorhanden ist.

**Revendications**

1. Procédé continu pour la production de l'acide iso- ou téréphtalique, qui comprend l'oxydation, au moyen d'oxygène moléculaire, de méta- ou paraxylène en présence d'un catalyseur comprenant du brome ou un composé contenant du brome isolément ou conjointement avec un métal lourd catalytique, caractérisé en ce que la réaction est exécutée à une température comprise dans l'intervalle de 200 à 300°C dans un milieu de réaction essentiellement aqueux et est initiée par addition, uniquement à son début, d'acide isophtalique ou d'acide téréphtalique comme agent solubilisant respectif pour le méta- ou le para-xylène.

2. Procédé suivant la revendication 1, caractérisé en ce que la réaction est initiée en présence de 1 à 20%, en poids du milieu de réaction aqueux, d'acide isophtalique ou téréphtalique.

3. Procédé suivant l'une des revendications précédentes, caractérisé en ce qu'il est exécuté à une température de l'intervalle de 210 à 260°C.

4. Procédé suivant l'une quelconque des revendications précédentes, caractérisé en ce que la métal lourd est le manganèse éventuellement conjointement avec le cobalt et/ou le nickel.

5. Procédé suivant l'une quelconque des revendications précédentes, caractérisé en ce que du brome, du bromure d'hydrogène ou un bromure d'un métal lourd est présent.

6. Procédé suivant l'une quelconque des revendications précédentes, caractérisé en ce que de l'iode ou un composé contenant de l'iode est présent comme activateur du catalyseur.